# EUROPEAN PATENT APPLICATION

(11) **EP 3 196 836 A1**
(43) Date of publication of application: **26.07.2017**
(21) Application number: 15842879.7
(22) Date of filing: 17.09.2015
(51) Int. Cl.: G06Q 50/24

(54) **SYSTEM FOR PREDICTING RISK OF ONSET OF CEREBROVASCULAR DISEASE**

(30) Priority: 19.09.2014 JP 2014191401
(71) Applicant: Shinano Kenshi Co., Ltd., Ueda-shi, Nagano 386-0498 (JP)
(72) Inventor: OKUBO, Masashi, Ueda-shi Nagano 386-0498 (JP); NAKAMURA, Hiroyuki, Ueda-shi Nagano 386-0498 (JP)
(74) Representative: Schäfer, Matthias W.
(86) International application number: PCT/JP2015/076589
(87) International publication number: WO 2016/043299

(57) **Abstract**

Attempts have been made in the past to predict the risk of onset of a disease from biological information, but, in practical terms, it is difficult to continuously acquire biological information on a regular basis, and there is no increase in the accuracy of predicting variations in the risk of onset of a disease based only on the acquired biological information. A compact pulse wave information acquisition element is fitted to a measurement subject, information is continuously acquired on a regular basis, and the risk of onset of cerebrovascular disease is predicted from fluctuations in blood pressure and pulse waveform on the basis of the pulse wave information continuously acquired on a regular basis.

## Description

### {Technical Field}

The present invention relates to a system for predicting a risk of onset of a particular disease and predicting a change in a risk of onset of a disease to give a warning.

### {Background Art}

It has traditionally been recommended to measure biological information such as a blood pressure and a heart rate intermittently, in addition to at visiting a medical institution, for the purpose of preventing the onset of a severe disease such as a cerebrovascular disease. In particular, hypertension has received attention as it can cause the onset of many diseases.

Those who have a low risk of onset of a disease keep track of a medium and long term change in such biological information by a blood pressure measurement, an electrocardiogram measurement and the like performed several times a year in a medical examination or the like. The measurement of the biological information performed for such purpose is a simple one and one that measures a temporary state of the blood pressure.

However, the measurement of such temporary state of the blood pressure is one that determines a risk of onset of a disease or a health condition on the basis of how much the biological information obtained at the time deviates from so to speak a standard value and thus is not something that determines the risk of onset of a disease by continuously observing a change in the biological information of a subject himself who is a subject of the measurement.

The biological information such as the blood pressure needs to be measured more frequently as aging or deterioration in a cardiovascular function advances. A blood vessel is significantly affected in particular so that, for an elderly person, it is important to keep track of a change in the blood pressure value. Accordingly, there is required a measurement technique that frequently measures the blood pressure value.

It is required in the blood pressure measurement to reduce a burden on a measurer (who is also a subject) measuring his own condition where, for such purpose, PTL 1 and PTL 2 disclose a technique of reducing the burden on the measurer in the blood pressure measurement.

However, even the use of these disclosed techniques cannot reduce a psychological burden on the measurer that he needs to consciously perform each of several blood pressure measurements a day with the understanding of necessity of the measurement.

The applicant has therefore proposed a biological information reading device in Japanese Patent Application No. 2014-153171 (PTL 3) that is compact and lightweight as well as formed into a flexible thin film. The biological information reading device is stuck on the subject or the like to be able to read biological information continuously at all times. Such biological information reading device can continuously measure the blood pressure at all times so that the subject is released from the constraint of the measuring.

### {Citation List}

### {Patent Literature}

{PTL 1} : JP 2011-200262 A
{PTL 2}: JP H01-214339 A
{PTL 3}: WO 2016/017579 A1

### {Summary of Invention}

### {Technical Problem}

### (Problem of knowledge about biological information)

There exists a further problem even when the measurer can measure the biological information continuously at all times without the psychological burden. When taking the blood pressure as an example in a case where a blood pressure value is measured frequently due to advanced hypertension, the measurer does not always have the knowledge of how a chronological change in the blood pressure value obtained in every measurement acts on various diseases. The knowledge rather belongs to the realm of a specialist such as a doctor, and thus it is difficult for an ordinary person to predict an urgent increase in the risk of onset of a disease even when he knows the change in the blood pressure value.

### (Problem of individual difference)

An individual physical condition of the subject also affects the change in the risk of onset of a disease.

A physical feature (such as age, gender, height, and weight) and a medical feature (such as presence or absence of an established disease and presence or absence of a congenital disease) of the subject are important elements, for example.

These elements affect the risk of onset of a disease so that similar biological information acquired under the same environment is evaluated differently for each subject being subjected to the measurement.

### (Problem of oversight in examination)

Moreover, a conventional examination is performed only in a specific short period of time of a day whether it is a blood pressure measurement or an electrocardiogram measurement, and thus has often missed a sign of symptoms of various diseases in its very early stage.

In the case of the electrocardiogram, for example, it is almost certainly impossible to find an initial symptom where an irregular heartbeat or the like occurs irregularly on rare occasions in a medical examination or the like that is performed once a year. Young people in particular do not usually wish to take the electrocardiogram measurement over an extended period of time except for those who have extremely high health awareness or those who are worried about a condition caused genetically, where there exists a significant problem that a sign of a disease that can possibly be found if the measurement is taken continuously over the extended period of time cannot be found at the time of the examination. Note that so called masked hypertension also belongs to this problem.

### (Problem of difficulty in realizing avoidance behavior)

In addition, for many diseases, a patient does not notice any symptoms until the disease advances to a severe condition so that, even when he acquires information such as a contraindication to the disease or a caution against a particular situation from a primary physician or the like, the patient being unable to directly realize any effects of paying attention to those information is not motivated to keep paying attention, thereby encountering a problem that he comes down with the same disease repeatedly or the like to waste medical resources or the like as a result.

Specifically, the problem is that it is difficult for the patient to grasp a general view of the degree of the risk of onset of a disease and a change in the risk thereof in a long term perspective and that it is difficult to grasp how much the change in the risk of onset of a disease is reduced by an individual avoidance behavior (such as "avoiding sudden strenuous exercise" or "not entering cool environment suddenly" in the case of hypertension) when the avoidance behavior is performed by the patient.

### {Solution to Problem}

The present invention has been made in order to solve the aforementioned problems.

That is, in view of the aforementioned circumstances, the present invention aims to continuously acquire biological information, particularly pulse wave information, at all times, obtain a change in the blood pressure and/or a change in the pulse wave pattern such as information on an irregular heartbeat from the pulse wave information that is acquired continuously, and predict the risk of onset of a disease, particularly a cerebrovascular disease, on the basis of the change.

The present invention includes: a biological information acquisition unit for continuously acquiring pulse wave information as biological information of a subject; an acquired information storage unit for storing the pulse wave information acquired by the biological information acquisition unit; a disease onset risk prediction unit for predicting a risk of onset of a cerebrovascular disease of the subject on the basis of the pulse wave information being stored; and an alarm output unit for issuing an alarm about a risk of onset of a disease on the basis of the prediction by the disease onset risk prediction unit, wherein the disease onset risk prediction unit predicts the risk of onset of the cerebrovascular disease on the basis of a change in each of a blood pressure and a pulse wave pattern based on the pulse wave information acquired continuously.

The prediction of the risk of onset of a disease is to determine whether or not the change in each of the blood pressure and the pulse wave pattern being acquired exceeds a normal range, for issuing an alarm when the change exceeds the normal range.

Moreover, the determination on whether or not the change in each of the blood pressure and the pulse wave pattern exceeds the normal range can be made by employing a statistical determination method that determines whether the change in each of the blood pressure and the pulse wave pattern being acquired is in the normal range or an abnormal range.

Furthermore, it can be determined that the risk of onset of a disease is high when a blood pressure value and a pulse rate acquired from the subject deviate to a large extent from a range of correlation, on the basis of a correlation between the blood pressure and the pulse rate acquired from the pulse wave pattern.

Furthermore, one or a plurality of pieces of information among activity information, physical information, disease information and environmental information of the subject is acquired beforehand, so that the onset risk prediction unit can predict the risk of onset of a cerebrovascular disease on the basis of all or an arbitrarily selected piece of the activity information, the physical information, the disease information and the environmental information of the subject as well as the pulse wave information being acquired.

The cerebrovascular disease to be predicted may include an ischemic cerebrovascular disease and a hemorrhagic cerebrovascular disease.

### {Advantageous Effects of Invention}

According to the present invention, the information on the change in the blood pressure and/or the pulse wave pattern is obtained on the basis of the pulse wave information acquired continuously at all times to be able to predict the risk of onset of the cerebrovascular disease and, when the risk is high, notify of the risk as a warning.

This can solve the aforementioned problems of the knowledge about the biological information, the individual difference, the oversight in the examination, the difficulty in realizing the avoidance behavior and the like.

### {Brief Description of Drawings}

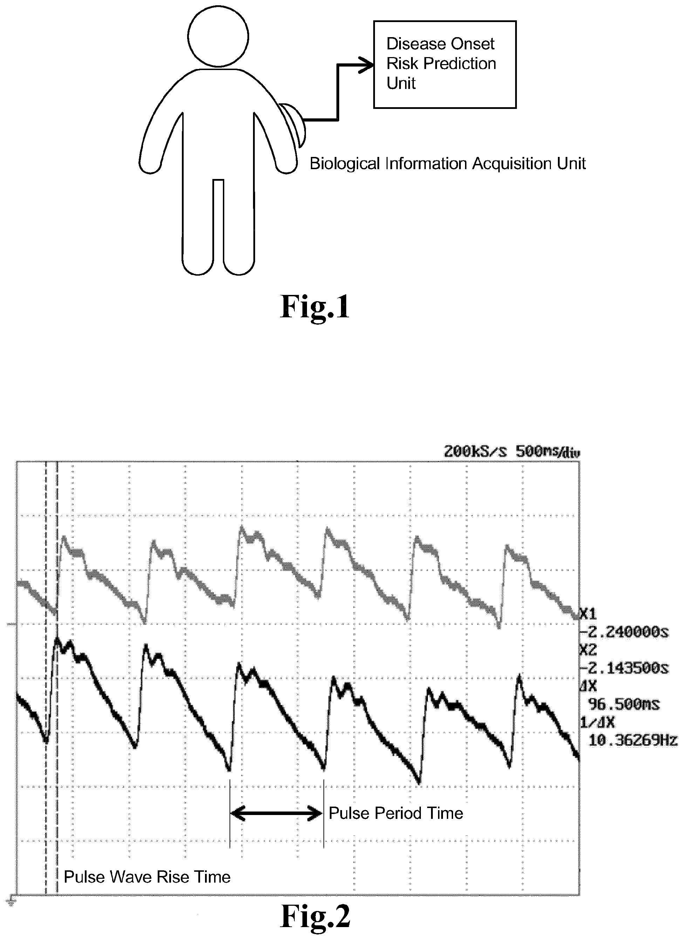

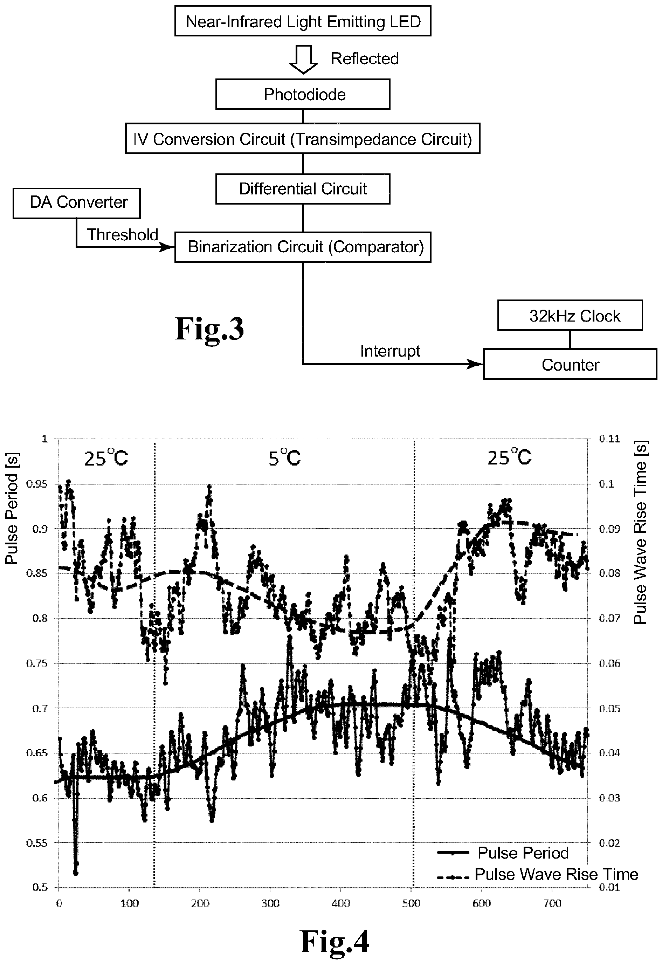

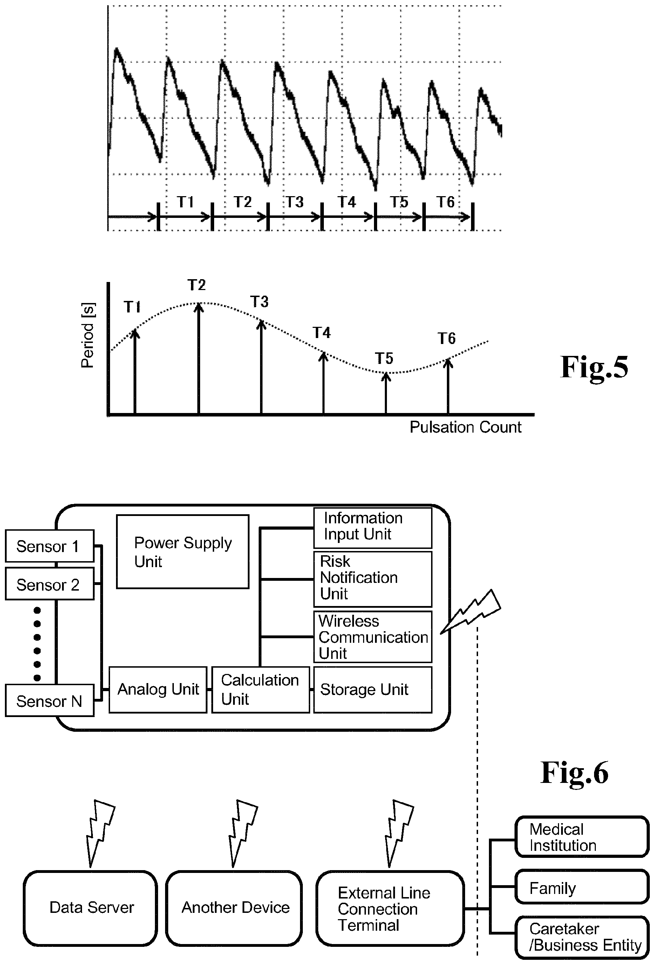

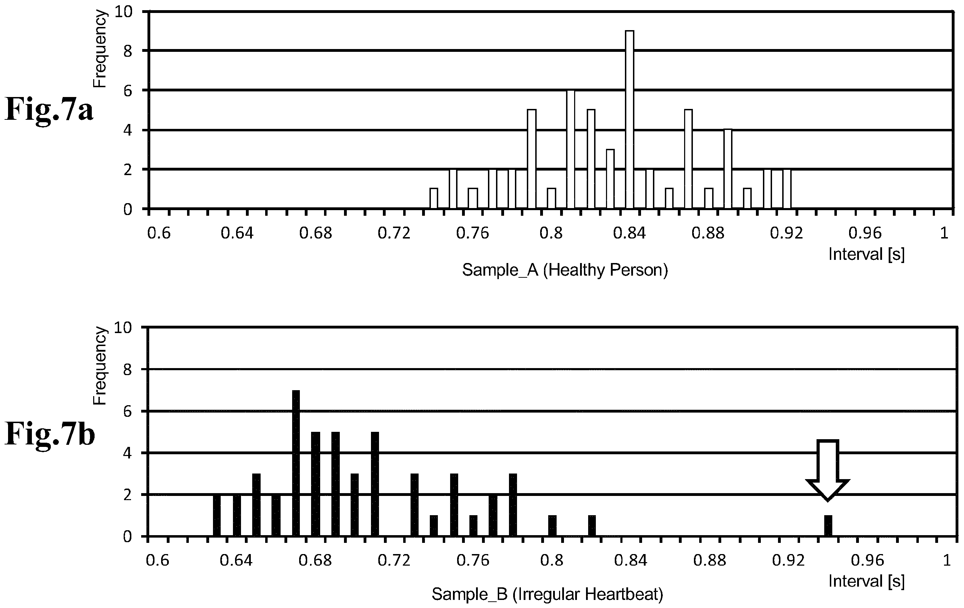

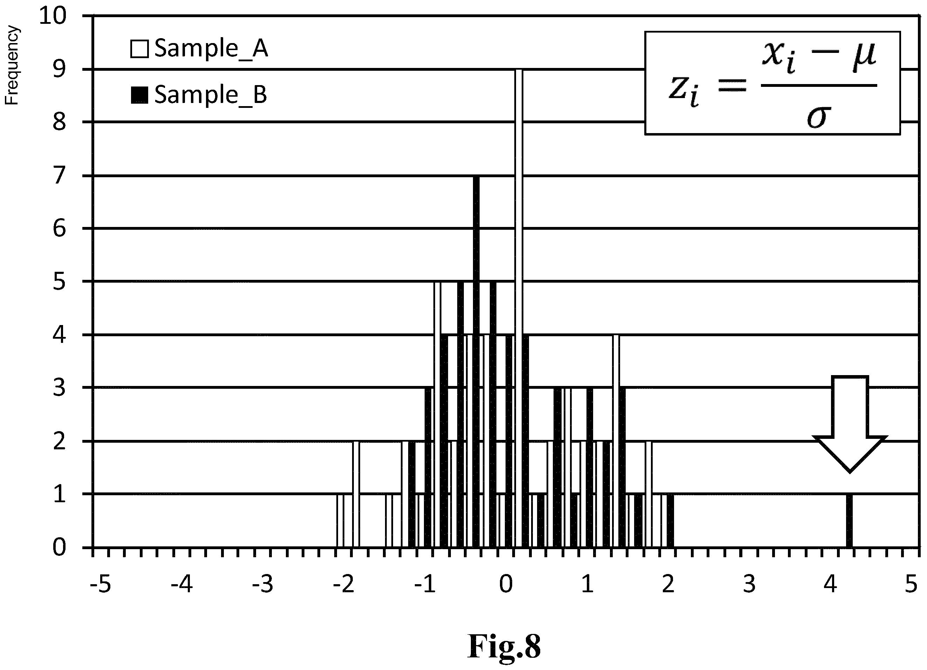

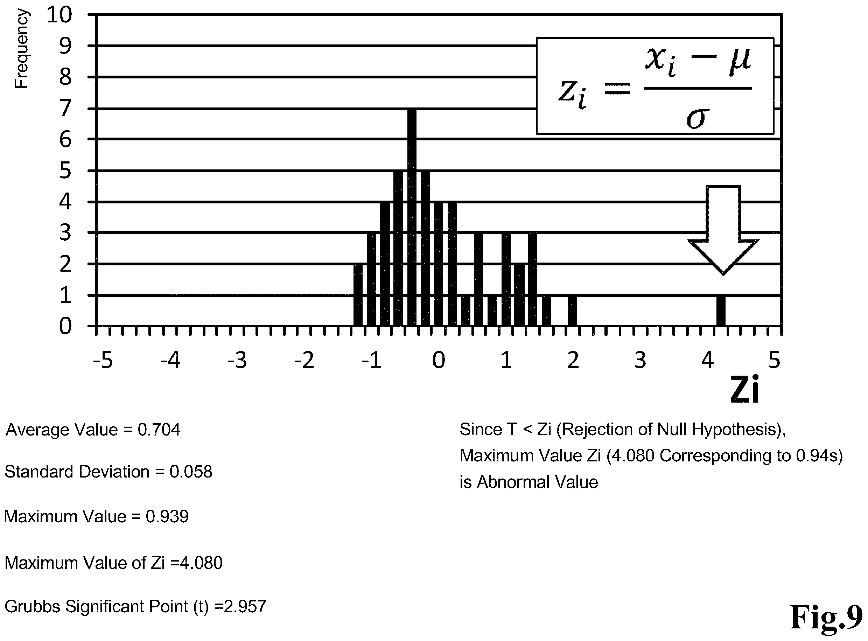

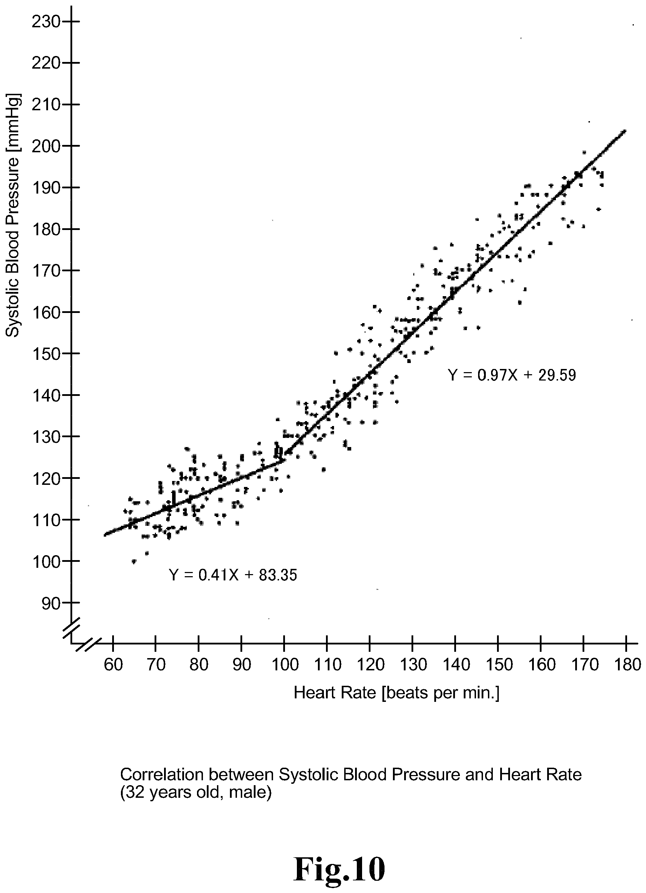

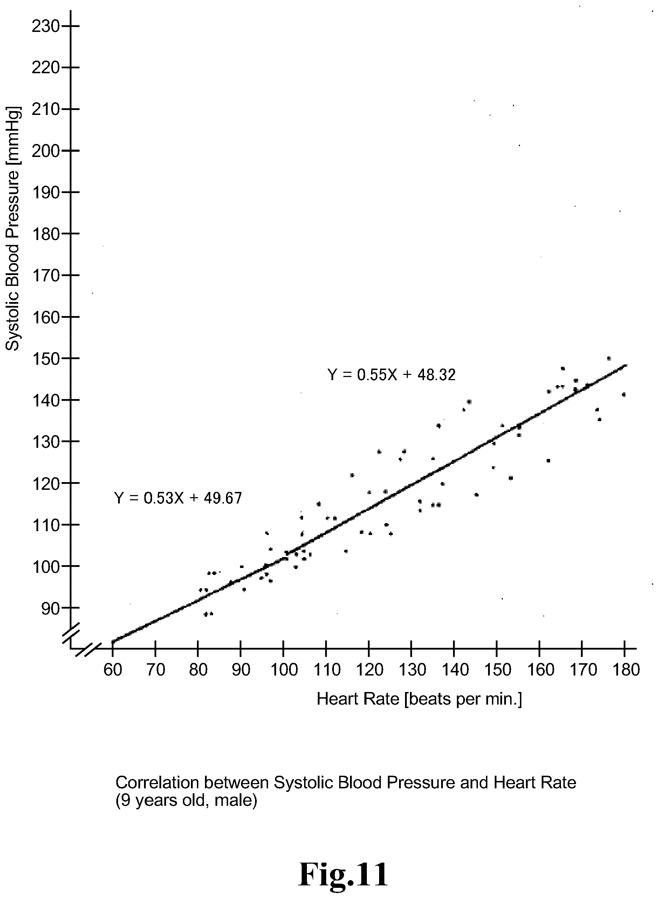

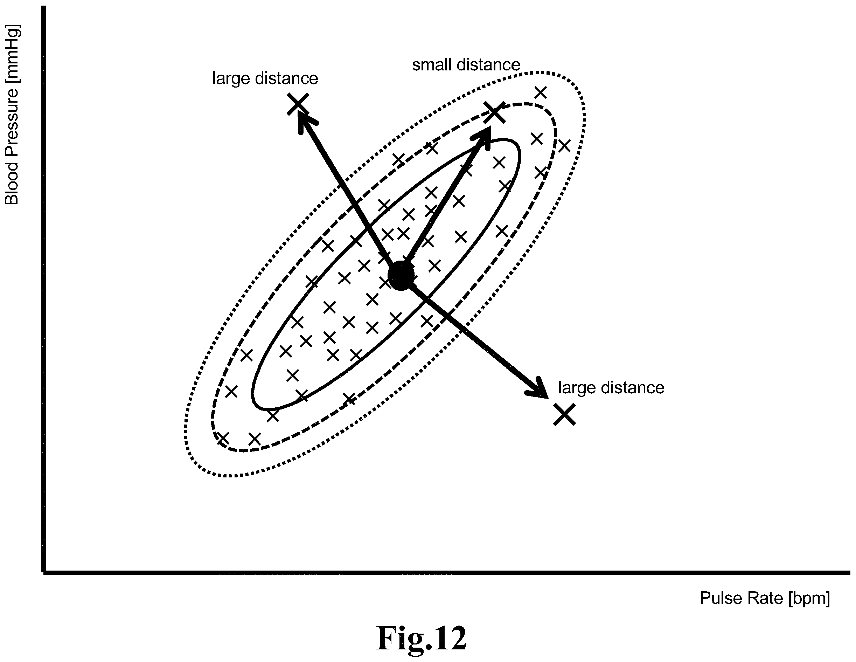

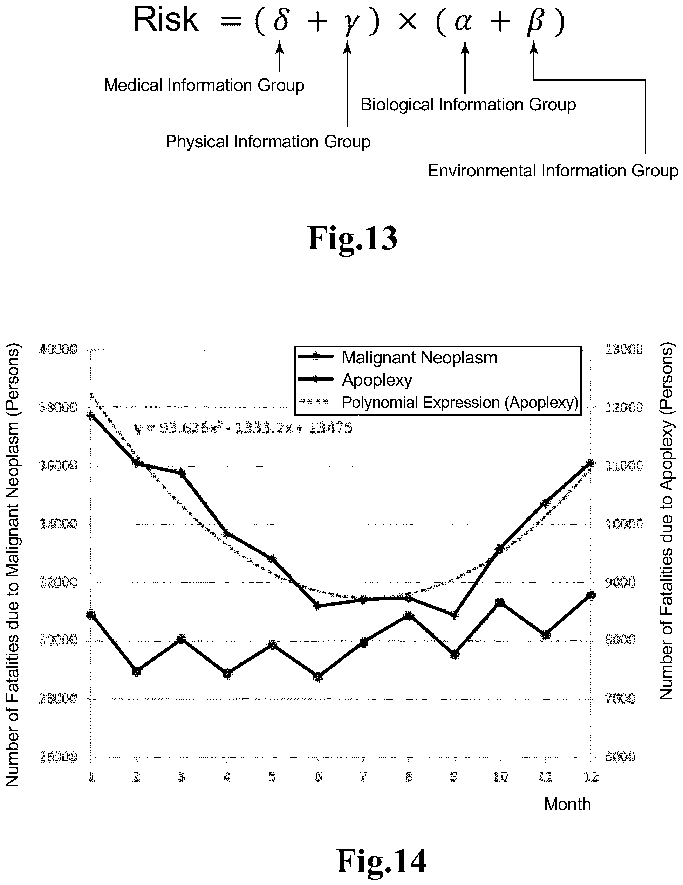

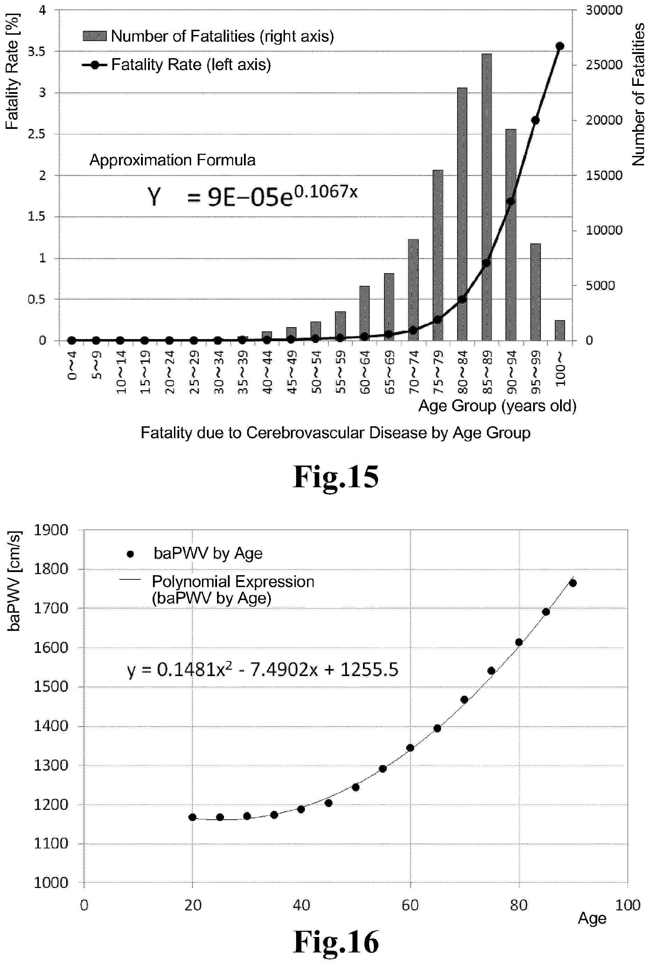

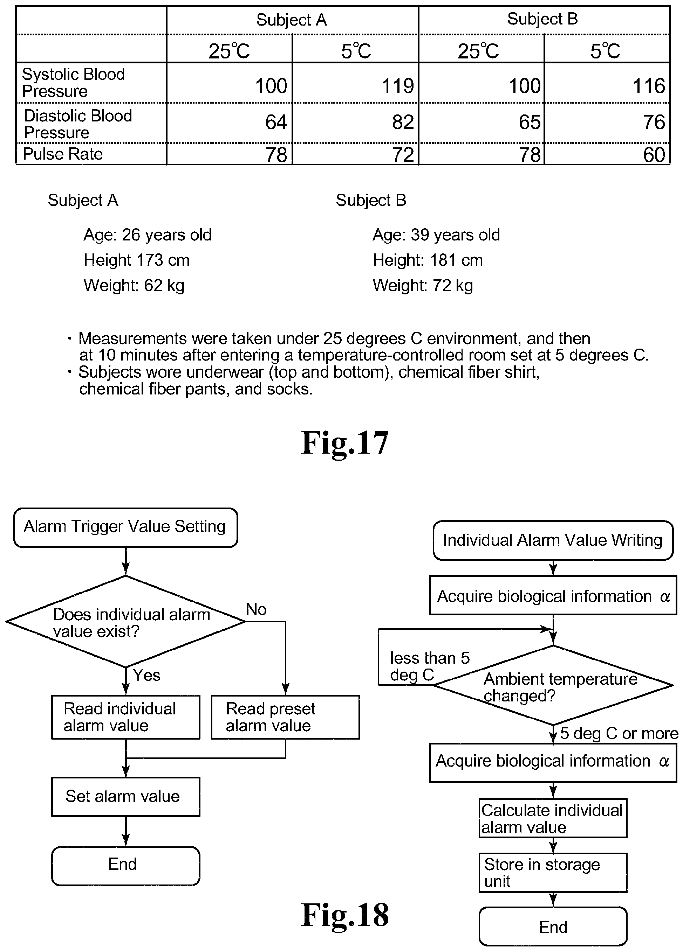

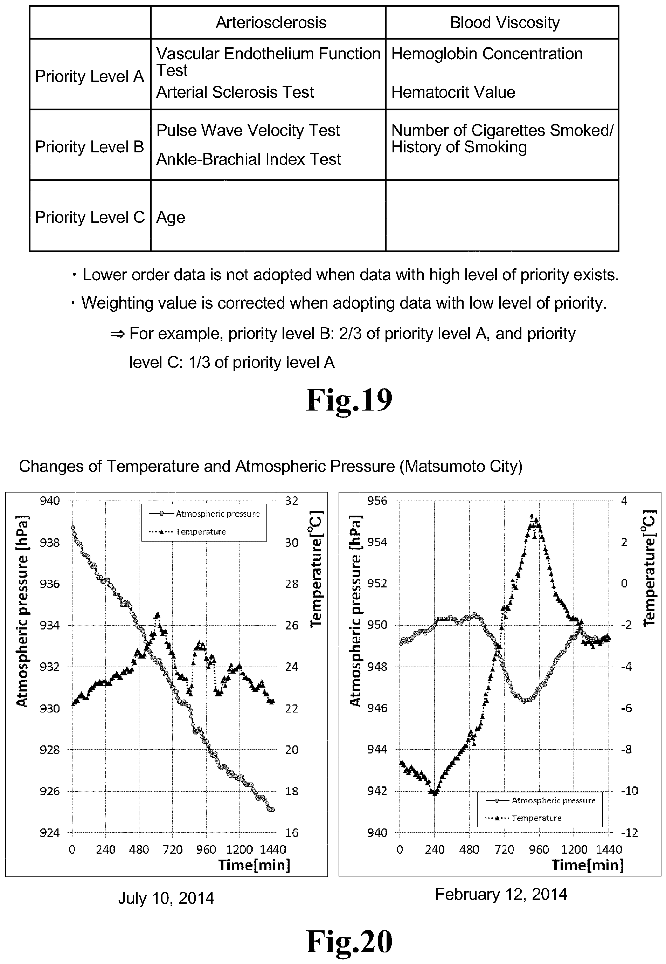

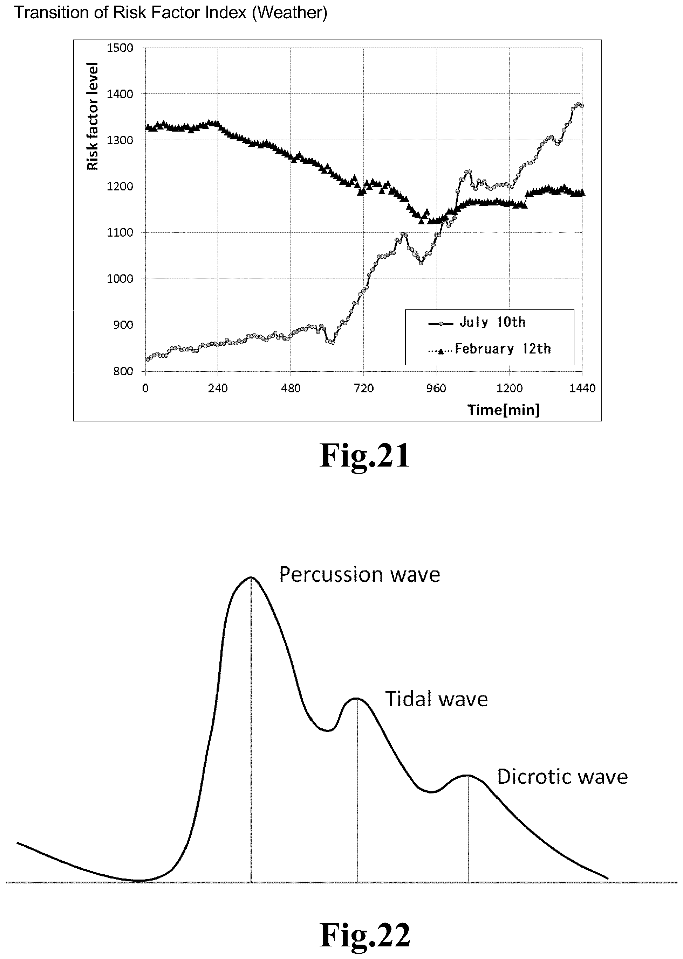

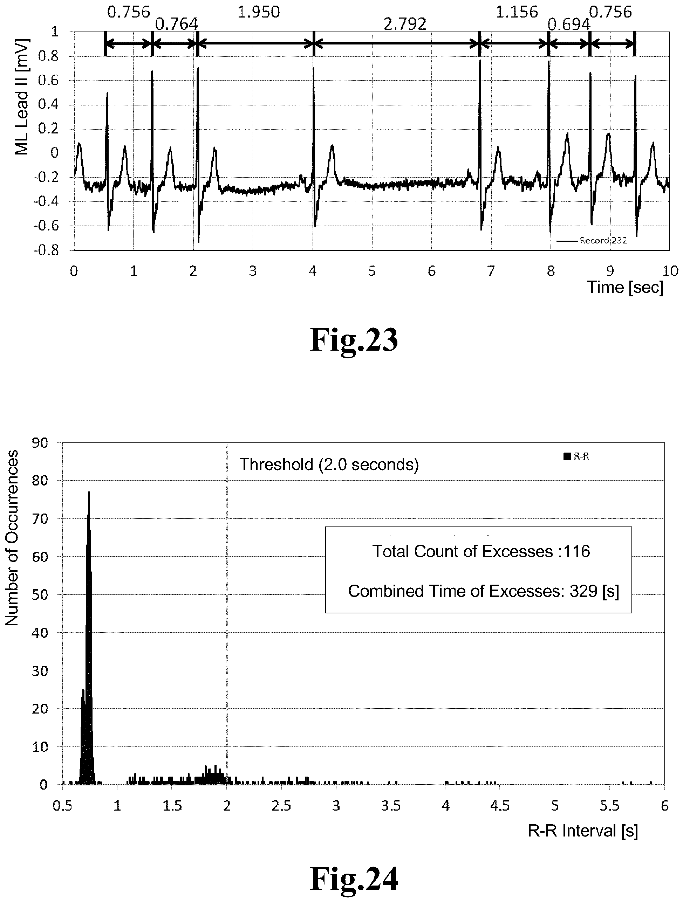

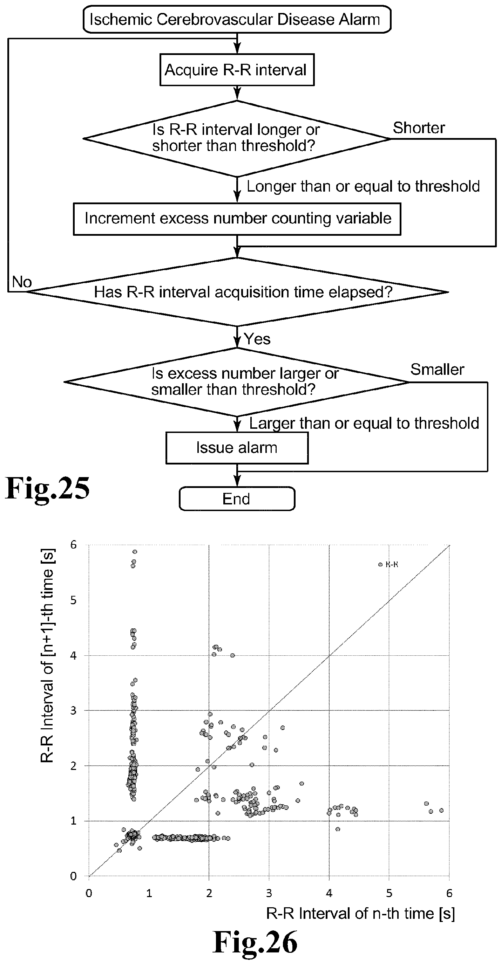

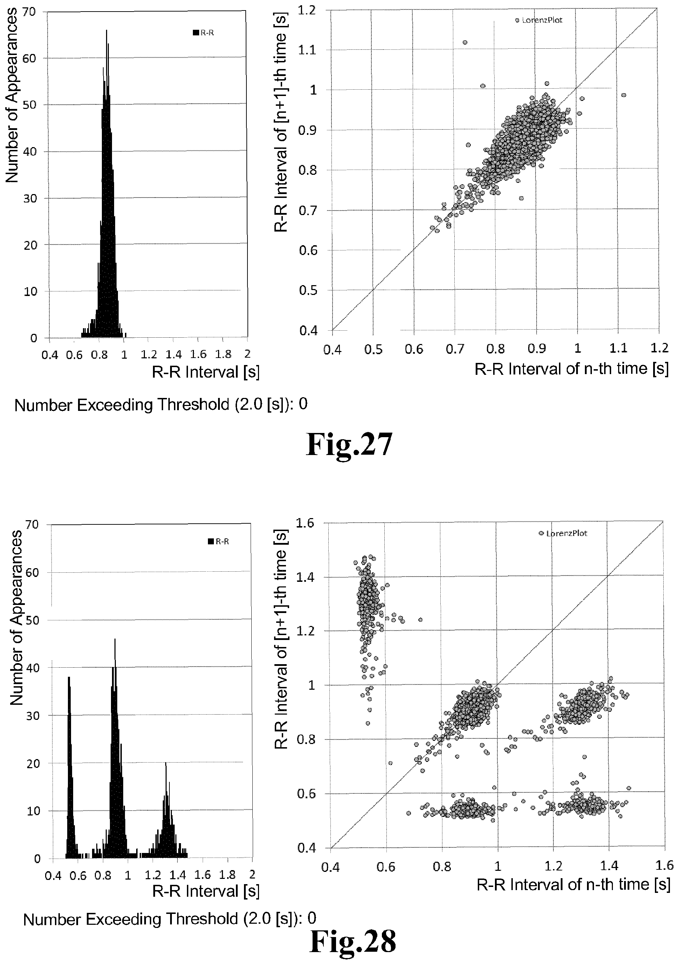

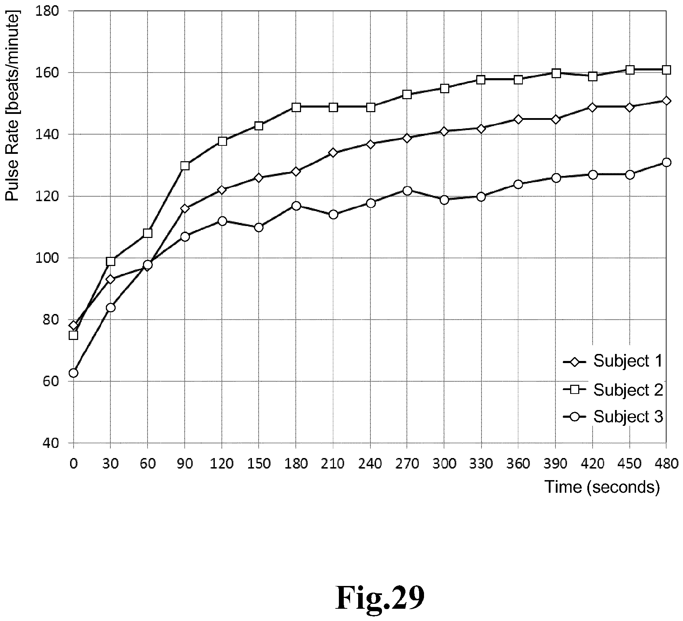

{Fig. 1} Fig. 1 is a diagram illustrating an example of a usage pattern of the present invention.
{Fig. 2} Fig. 2 is a diagram used to describe a pulse wave pattern handled in the present invention.
{Fig. 3} Fig. 3 is a diagram used to describe a procedure of reading the pulse wave pattern in the present invention.
{Fig. 4} Fig. 4 is a diagram illustrating a relationship between a change in ambient temperature and the pulse wave pattern.
{Fig. 5} Fig. 5 is a diagram illustrating a fluctuation of the pulse wave pattern.
{Fig. 6} Fig. 6 is a block diagram illustrating an example of the usage pattern of the present invention.
{Fig. 7} Fig. 7 is a diagram representing a heartbeat time interval in the form of a histogram, where Fig. 7a illustrates data of a healthy person while Fig. 7b illustrates data of a person with an irregular heartbeat.
{Fig. 8} Fig. 8 is a diagram obtained by normalizing the data in Fig. 7.
{Fig. 9} Fig. 9 is a diagram used to describe an example in which a Smirnov-Grubbs test is applied to the data of the person with the irregular heartbeat in Fig. 7.
{Fig. 10} Fig. 10 is an example illustrating a correlation between a blood pressure and a heart rate when one exercises.
{Fig. 11} Fig. 11 is another example illustrating the correlation between the blood pressure and the heart rate when one exercises.
{Fig. 12} Fig. 12 is a diagram illustrating a test using a Mahalanobis distance between a pulse and the blood pressure.
{Fig. 13} Fig. 13 is a diagram illustrating an expression for a concept of calculating a risk of onset of a disease.
{Fig. 14} Fig. 14 is a diagram illustrating the number of fatalities due to a disease in each month.
{Fig. 15} Fig. 15 is a diagram illustrating the number of fatalities due to a cerebrovascular disease by age.
{Fig. 16} Fig. 16 is a diagram illustrating a correlation between age and a degree of arteriosclerosis.
{Fig. 17} Fig. 17 is a diagram illustrating a change in the blood pressure and pulse with respect to a change in environmental temperature.
{Fig. 18} Fig. 18 is a diagram illustrating an example of an algorithm for outputting a warning.
{Fig. 19} Fig. 19 is a diagram illustrating an example of a level of priority of biological information used.
{Fig. 20} Fig. 20 is a diagram illustrating an example of a change in temperature and atmospheric pressure in the city of Matsumoto, Nagano prefecture.
{Fig. 21} Fig. 21 is a diagram illustrating an example of a risk of onset of a disease based on the temperature and atmospheric pressure illustrated in Fig. 20.
{Fig. 22} Fig. 22 is a diagram used to describe a characteristic point in the pulse wave pattern.
{Fig. 23} Fig. 23 is a diagram illustrating an example of the irregular heartbeat shown in an electrocardiogram.
{Fig. 24} Fig. 24 is a diagram illustrating an example of a histogram of an R-R interval.
{Fig. 25} Fig. 25 is a diagram illustrating an example of a warning output algorithm for an ischemic cerebrovascular disease.
{Fig. 26} Fig. 26 is a diagram illustrating an example of a Lorenz plot.
{Fig. 27} Fig. 27 is a diagram illustrating a histogram and a Lorenz plot based on an R-R interval different from that in Fig. 26.
{Fig. 28} Fig. 28 is a diagram illustrating a histogram and a Lorenz plot based on an R-R interval different from that in Fig. 27.
{Fig. 29} Fig. 29 is a diagram illustrating an example of duration of a stepping exercise and a change in the pulse.

### {Description of Embodiments}

Embodiments of the present invention will now be described with reference to the drawings.

### (First embodiment)

An overview of a system according to an embodiment of the present invention will be described with reference to Figs. 1 to 6.

As illustrated in Fig. 1, a system including a biological information acquisition unit and a disease onset risk prediction unit to predict a risk of onset of a cerebrovascular disease according to the present invention is attached to a region such as near a radial artery suited for acquiring pulsation of a blood vessel of a subject (a measurer himself) so that the biological information acquisition unit acquires a pulsating waveform of the blood vessel being a piece of biometric information of the subject. When a hemorrhagic cerebrovascular disease is targeted in particular, a site near the blood vessel in the head such as a superficial temporal artery or a facial artery is suitable for acquiring the pulsation of the blood vessel.

Note that the pulsating waveform of the blood vessel is described as a pulse wave pattern.

The biological information acquisition unit has a projector and an optical receiver.

Note that the projector and the optical receiver used herein are representative means of acquiring blood vessel pulsation information, where equivalent information can also be acquired by a piezoelectric element, a microphone, a pressure/load sensor, or a bioimpedance sensor.

Fig. 2 illustrates an example of the pulse wave pattern acquired by the biological information acquisition unit illustrated in Fig. 1. Two waveforms (top and bottom) illustrated herein are acquired from the same person under different conditions.

As the two top and bottom waveforms illustrated in Fig. 2 are different from each other, even the blood vessel pulsation information acquired from the same subject changes every moment by an activity of an internal circulatory system according to mental and physical conditions.

Moreover, it is widely known that an initial rise time of a period of the pulse wave illustrated in Fig. 2 is correlated with a blood pressure value and that a respiratory condition as well as a Mayer wave cause a change in a pulse period (= a pulse wave period), where much biological information can be acquired by processing the blood vessel pulsation information.

Fig. 3 illustrates an example of the flow of processing the blood vessel pulsation information.

The projector formed of an LED emitting near-infrared light emits (near-infrared) light, which is reflected off the blood vessel so that the reflected light is detected by the optical receiver formed of a photodiode and then converted into a voltage signal in an IV conversion circuit.

Next, a differential circuit as disclosed in JP H10-295657 A, for example, is used to convert the signal into a velocity pulse wave to facilitate acquisition of the pulse wave rise time and the pulse period time.

The velocity pulse wave output from the differential circuit is binarized by a comparator, and the binarized signal is input to an interrupt input unit of a counter operating with a predetermined clock.

As a result, an interval until an interrupt occurs is acquired as a count value of the clock. The pulse wave rise time and the pulse period time can thus be acquired.

Fig. 4 illustrates a result of continuously monitoring the pulse wave rise time and the pulse period time when the present system is attached to a subject who moves from a room temperature (25 degrees Celsius) environment into a low temperature (5 degrees Celsius) environment.

A reaction of a blood circulatory system against low temperature mainly includes an increase in the blood pressure and a decrease in the heart rate, where one can also read from the result in Fig. 4 a reduction in the pulse wave rise time (increase in the blood pressure) and extension of the pulse period time (decrease in the heart rate ≈ pulse).

The upper part of Fig. 5 illustrates a concept of a period time of a pulse obtained from the pulse wave pattern. Time is set as the unit on a horizontal axis. Treating T1 to Tn as periods of continuous pulse waves, one obtains a fluctuation by performing a frequency analysis on how the period time changes in each pulsation, as illustrated in the bottom part of Fig. 5. The fluctuation indicates a degree of activity of the autonomic nerve.

Fig. 6 is a block diagram illustrating an overview of the present system.

Sensors 1 to N represent N pieces of sensors. The sensor specifically corresponds to the biological information acquisition unit and an ambient information acquisition unit. N may be equal to one, two or larger. A signal and/or information acquired by the sensor is subjected to analog-to-digital (A/D) conversion by an analog unit and converted into a digital value.

The digital value is subjected to calculation by a calculation unit, stored in a storage unit or, when it is determined that a warning needs to be issued on the basis of the calculation result, the warning is issued through a risk notification unit.

An information input unit is an input device existing on the present system and used to perform an input operation directly. Specifically, the information input unit is a keyboard, a mouse, and/or a touch screen including an input information checking unit such as an LED or LCD.

A wireless communication unit mainly aims at notifying a result of determination made by the calculation unit of the present system to the outside of the system. The risk notification unit mainly issues the warning to a measurer, whereas the wireless communication unit transfers data acquired by the sensor or the result of determination made by the calculation unit to the outside in order for such data or result to be used as material for further service.

The storage unit in the system may run short of capacity when the biological information of the subject is measured continuously over an extended period of time, for example, in which case the wireless communication unit can be used to transfer the information to the outside in order to compensate for the shortage.

Moreover, the warning issued on the basis of the determination made by the calculation unit can be transferred to a medical institution, a relative, a municipality (such as a welfare commissioner), a care facility frequented by the subject and the like at a remote location.

Here, there will be described how the present embodiment performs the prediction on the risk of onset of the cerebrovascular disease.

An apoplexy being the cerebrovascular disease can be roughly classified into an ischemic cerebrovascular disease and a hemorrhagic cerebrovascular disease. The onset of such apoplexy is thought to be significantly contributed by a change in the blood pressure and a change in the pulse period, particularly the occurrence of the irregular heartbeat.

The irregular heartbeat is mainly caused by atrial fibrillation. This causes blood to be retained in the heart and coagulated by activation of a blood coagulation factor, thereby causing a thrombus to occur more easily. In addition, a plaque in a blood vessel is triggered by the occurrence of the irregular heartbeat and moves through the blood vessel. The plaque moving into the brain is thought to clog a cerebral blood vessel.

Moreover, a sudden increase in the blood pressure is thought to contribute significantly to the occurrence of the hemorrhagic cerebrovascular disease.

Accordingly, the risk of onset of the disease can be predicted by detecting the change in the blood pressure, particularly the increase in the blood pressure, and the occurrence of the irregular heartbeat and determining how much these values deviate from those in a normal state of the subject.

The pulse wave is acquired continuously in the present embodiment so that the change in the pulse wave can be determined separately for a factor changing in a short term, a factor changing in a medium term, and a factor changing in a long term. Accordingly, as for the blood pressure, there can be detected an increase in the blood pressure having the risk of causing the onset of a disease by considering a change in the blood pressure in one's life from wake-up to a daytime activity such as having a meal or bedtime. Moreover, a frequency of occurrence of the irregular heartbeat that does not normally occur is considered to be able to determine an increase in the risk of onset of a cerebral blood vessel disease.

There will now be described an example of a method of determining detection of the occurrence of the irregular heartbeat from data on a time interval of the pulse wave, namely a heartbeat interval.

Fig. 7 is a diagram representing data on the heartbeat interval in the form of a histogram, where Fig. 7a illustrates data of a healthy person while Fig. 7b illustrates data of a person with the irregular heartbeat. Here, a part (approximately 0.94 seconds) pointed by an arrow in Fig. 7b indicates a target to be detected as the irregular heartbeat. Fig. 8 illustrates a frequency distribution superposed on a normalized value of the value in Fig. 7.

Fig. 9 is a diagram illustrating a determination on whether the data (approximately 0.94 seconds) pointed by the arrow is an abnormal value by performing a Smirnov-Grubbs test on the data having the irregular heartbeat in Fig. 8 described above where, when the test is performed for the part pointed by the arrow, namely Zi = (χi - µ)/σ, an average value equals 0.704, a standard deviation equals 0.058, the maximum value equals 0.939, the maximum value of Zi equals 4.080, and a Grubbs significant point (t) equals 2.957, so that with t < Zi, the maximum value Zi (4.080 corresponding to 0.94 seconds) can be determined as an abnormal value to be able to detect the irregular heartbeat from the pulse wave measured continuously.

It is also known that there is a correlation between the blood pressure and the heart rate. Figs. 10 and 11 illustrate the correlation between the systolic blood pressure and the heart rate when one exercises, where Fig. 10 illustrates an example of a 32 year-old male while Fig. 11 illustrates an example of a 9 year-old male (Memoirs of Osaka Kyoiku University, Vol. 23, Ser. 3, 1974). Correlation information between the blood pressure and the pulse rate of an individual subject can be obtained by recording the blood pressure (estimated from the pulse wave pattern) in association with the heart rate (calculated from a period of the pulse wave pattern) continuously for the subject on the basis of the correlation between the blood pressure and the heart rate.

A large number of this associated records are sampled beforehand to allow for a comparison between the "blood pressure value estimated from the pulse wave pattern" and the "blood pressure value estimated from the pulse rate correlation" to be able to check for separation therebetween. When the separation is large, one can determine a condition in which "the subject is in a condition different from that in the sample acquired in the past".

This "associated record of the blood pressure and the pulse rate" is used to be able to find a Mahalanobis distance between a "specific sample" and a "group made up of other samples", so that a threshold for an outlier test is set with respect to the Mahalanobis distance as illustrated in Fig. 12 to be able to check whether the specific sample is abnormal and detect a degree of the abnormality. As a result, the occurrence of the abnormality with the blood pressure of the subject can be detected.

Therefore, with regard to the blood pressure and the irregular heartbeat, the acquired pulse wave can be used to detect a large deviation of the blood pressure of the subject from a normal range and the occurrence of the abnormal irregular heartbeat so that, on the basis of this condition, one can determine that the blood pressure and the pulse have changed from a normal state to an abnormal state and that the risk of onset of the cerebrovascular disease is increased.

With regard to the irregular heartbeat as well, information on a frequency of occurrence of the individual subject exists so that, on the basis of the information on the frequency of the occurrence of the irregular heartbeat of the individual subject, one can determine whether the irregular heartbeat occurring is in a normal range or outside the normal range. One can thus determine the increase in the risk of onset of the cerebral blood vessel disease, namely a change in the risk thereof, on the basis of the data on both the blood pressure and the pulse (heart rate).

In this case, the determination is not made on an event that the blood pressure value or the pulse rate is too high or too low, namely on the magnitude of the value, but is made on whether the change in the blood pressure or the pulse rate continuously acquired deviates from a normal correlation on the basis of the change in the blood pressure or the pulse rate.

In addition to the aforementioned determination using the Mahalanobis distance, the determination can be made by using a statistical test method or the like as the method of determining whether or not a measured value deviates from the normal range.

The present application measures the pulse wave continuously at all times to be able to predict the risk of onset of a disease from an abnormal increase in the blood pressure and sudden occurrence of the irregular heartbeat that are not normal changes in the blood pressure and pulse among changes in the blood pressure and pulse that change over time unlike the blood pressure and electrocardiogram information measured intermittently.

Here, an interpretation of the term pulse wave pattern will be described.

Fig. 22 illustrates a characteristic of the shape of the pulse wave pattern as will be described later in detail.

The pulse wave pattern of a healthy person generally has the shape as illustrated in the figure, where the pulse wave pattern is characterized by a mutual relative positional relation among peaks of a "percussion wave", a "tidal wave", and a "dicrotic wave" and a feature point such as a zero crossing point. A change in the pulse wave pattern thus causes a change in the relative positional relation. Therefore, one can grasp the change in the pulse wave pattern by finding the relative positional relation of the pulse wave pattern.

However, the term pulse wave pattern in the present application also includes a concept other than the general concept of the waveform described above.

As one belonging to a micro level, for example, a fast Fourier transform (FFT) may be performed on the waveform of a first pulse wave to obtain a frequency characteristic, which can then be used to grasp the waveform, or standard pulse wave pattern data may be set in advance to obtain a correlation value with respect to the data so that the value can be used to grasp the waveform as well. The same can be said for a time width and an amplitude of the pulse wave.

Ones belonging to a macro level include, for example, the pulse wave period, a Lorenz plot and the like based on a mutual relationship among continuous pulse wave patterns as well as the pulse rate, a fluctuation of the pulse wave period and the like based on the pulse wave pattern included in a certain period.

It is needless to say that a plurality of pulse wave patterns and the period become clear as a spectrum by obtaining the frequency characteristic while targeting a section including the plurality of pulse waves and that a change can be more easily grasped in chronological order by observing the spectrum in a waterfall display. Moreover, the pulse wave pattern may be evaluated on the basis of statistical data based on data acquired and accumulated from an individual wearer or statistical data on a particular group to allow the change in the waveform to accurately affect the prediction of the risk of onset of a disease.

### (Second embodiment)

Here, prediction of a change in a risk of onset of a disease will be described.

As illustrated in an expression in Fig. 13, the risk is a function having medical information, physical information, biological information and environmental information as parameters. Interaction among the parameters is complex fundamentally. While the function expression is simplified in the present embodiment for the purpose of clarifying description, an expression used in actually predicting the change in the risk is not limited to this expression.

The biological information, environmental information, physical information and medical information will now be described again.

The biological information such as a blood pressure or a pulse per minute originates from a body of a measurer. Changing at all times, the information needs to be measured frequently or continuously to perform an accurate measurement.

The biological information also includes one that can be inferred from biological information obtained directly.

Putting these together, the biological information can be exemplified by a systolic blood pressure, a diastolic blood pressure, an autonomic nerve activity, a heart rate, a pulse rate, a respiration rate, a respiratory rhythm, a posture, a state of activity and the like.

The systolic blood pressure and the diastolic blood pressure are important for the purpose of knowing an adverse effect on the blood vessel based on a change in the blood pressure, and are particularly used as a factor for increasing a risk of a sclerosed artery, vasoconstriction, a rupture of an aneurysm and the like.

The autonomic nerve activity is important for the purposed of knowing vascular constriction and an increase in the heart rate caused by a sympathetic nerve activity. The sympathetic nerve activity as a factor for increasing the blood pressure causes an increase in a risk of onset of various diseases.

The heart rate and the pulse rate also serve as an index for determining a state of activity.

A heartbeat interval and a pulse period are important for the purpose of knowing an irregular heartbeat caused by atrial fibrillation or the like. When a long pulse interval causes blood to stagnate, a thrombus is generated to increase a risk of onset of an ischemic disease.

The respiration rate and the respiratory rhythm are important in identifying a site of apoplexy in the event thereof. They also serve as an index for determining the state of activity.

Note that as association between the affected site of the apoplexy and the type of the respiratory rhythm, it is known that a person experiences Cheyne-Stokes respiration when each of both cerebral hemispheres or a diencephalon is affected, central neurogenic hyperventilation when a lower mesencephalon or upper pons is affected, apneustic breathing when middle pons or lower pons is affected, cluster breathing when the lower pons or an upper medulla is affected, and ataxic breathing when a medulla is affected.

The posture refers to a standing position, a seated position, a recumbent position and the like and indicates a positional relation between the brain and the lungs as well as the heart with respect to the direction of gravity. A change in the posture becomes a factor for a change in the blood pressure and is important in identifying a factor changing the blood pressure.

The state of activity means a state of a body such as exercising, resting, sleeping and the like. The state of activity particularly becomes a factor for a change in the blood pressure and is important in identifying the factor.

The environmental information is a piece of information on the environment in which a measurer is placed, and includes temperature, humidity, atmospheric pressure, date and time and the like, for example. These pieces of information also have the predisposition to change at all times and thus need to be measured frequently or continuously considering the accuracy of predicting the change in the risk of onset of a disease.

The temperature is an important factor affecting thermoregulation, where rise and fall of the temperature itself as well as a change thereof per unit time and a total time for which the measurer is exposed to the temperature both affect the onset of a disease.

The humidity is a factor affecting the thermoregulation as with the temperature. High humidity inhibits the thermoregulation by perspiration as well as causes depletion of body water by a large amount of perspiration at high temperature, thereby affecting blood circulation as a result.

The atmospheric pressure is a factor for increasing the onset of the apoplexy, where it is statistically known that occurrence of a drop in the atmospheric pressure increases the incidence of the apoplexy.

The date and time means a date and a time.

The date is used for the purpose of knowing a medium to long term change in temperature, a change in gravity caused by the position of the moon (satellite) and the like. It is statistically known that there is a significant difference in the incidence of apoplexy in particular from month to month as illustrated in Fig. 8. The data is retrieved from a result of a demographic survey released by the Ministry of Health, Labour and Welfare.

Moreover, the change in gravity caused by the position of the moon (satellite) affects the inflow of blood into the head and thus affects the onset of a cerebral disease.

Furthermore, one can know the "type of the day" such as a day of the week or a national holiday on the basis of the date. A life pattern is different between a weekday and a holiday and thus affects the onset of a disease differently so that accuracy of prediction can be increased.

The time is used for the purpose of knowing at which stage of a life cycle of the day the measurer is. The time is particularly important in detecting morning hypertension after wake-up. The prediction can be made more accurately by using another state of activity together such as exercising and having a meal.

The physical information pertains to a body of the measurer but does not change over a short and medium term. Specifically, the physical information includes the age, gender, weight, height, presence or absence of smoking and drinking habits, and the like.

These pieces of information are input as preset values before activating the present system. The information such as the weight and age that change over a long term can be handled by correcting the preset values when one notices a change.

The age is an important factor for the incidence of particularly the cerebrovascular disease as illustrated in Fig. 15. The incidence increases exponentially as the age increases, so that one can understand there is a high risk of onset of the disease just because a person is of old age in a certain age group or higher, which indicates the importance of risk prediction itself. The data is retrieved from the demographic survey released by the Ministry of Health, Labour and Welfare.

Moreover, as illustrated in Fig. 16, a progress level of arteriosclerosis increases quadratically according to the age. The risk of onset of a disease is increased for a disease caused by the arteriosclerosis. The data is cited from the material on the age-specific pulse wave velocity published by Colin Medical Technology Corporation.

With regard to the gender, a vascular age (degree of arteriosclerosis) is different between males and females, where the vascular age of females in particular changes significantly before and after the menopause. Females in general have a lower value of the degree of arteriosclerosis than males of the same age due to the influence of female hormones before the menopause, but have the degree of arteriosclerosis increased to the same level as that of males for approximately 10 years after the menopause. Therefore, the gender is grasped together with the age to be used in the risk prediction.

With regard to the weight and height, an obesity condition can be known by calculating a BMI, from a value of which the risk of diabetes and hypertension can be known. The BMI of 25 to 30 is treated as overweight and 30 or higher as obese, for which a value of the risk of onset of a disease is increased.

The smoking and drinking habits become a factor for increasing the blood pressure and blood viscosity.

Smoking causes an increase in the blood pressure of approximately 20 mmHg and an increase in the number of red blood cells and the blood viscosity. A risk of mortality due to apoplexy is 2.8 times that of a non-smoker, statistically.

Moreover, excessive drinking causes an increase in the blood pressure and further causes reduced production of a blood coagulation factor as well as hypocholesterolemia with a liver dysfunction as a factor, whereby a risk of onset of the apoplexy is increased as a result. When drinking and smoking can be detected from the state of activity, these are used to predict the risk of onset of a disease on each such occasion.

The medical information is information related to medical care of the measurer known at the time and does not change over a short term. Specifically, the medical information includes a medical test value, a chronic disease and an established disease of the measurer himself and a close relative, where information involved in an increase in the risk of onset of a disease is weighted by adding a point according to the degree of the involvement.

The close relative used in this case includes a first-degree relative (mother and father, children, and brother and sister of the measurer) and a second-degree relative (grandparents, aunt and uncle, niece and nephew, grandchildren, and brother and sister whose one parent is different).

The medical test value includes a blood glucose level (including HbA1c), a blood lipid level, blood viscosity, a degree of arteriosclerosis, a diagnostic result of thrombus, and DNA testing.

It is confirmed by 12 years of follow-up surveys by National Cancer Center that the blood glucose level increases the risk of onset of cerebral infarction of a diabetes patient and can thus be used in the risk prediction.

An increase in the blood lipid level is a main factor for damaging an inner wall of an artery and causing atherosclerosis, and can also be used in the risk prediction.

The blood viscosity can be known by blood test items including a red blood cell count, hemoglobin concentration and a hematocrit, and can be used to predict the risk of an ischemic cerebrovascular disease.

The degree of arteriosclerosis can be obtained by a vascular endothelium function test, an arterial sclerosis test, the pulse wave velocity and an ankle-brachial index test, and can be used to predict the risk of a hemorrhagic cerebrovascular disease.

The diagnostic result of thrombus can be obtained by CT, MRI, ultrasonography and angiography, and can be used to predict the risk of the ischemic cerebrovascular disease.

With regard to the DNA testing, it is known by research findings published in The Lancet Neurology that a person holding DNA in a sub-haplogroup K of mitochondrial DNA has a remarkably lower risk of onset of apoplexy compared to a person holding DNA in another haplogroup.

The chronic disease and established disease are weighted by points as follows, for example: a past history of apoplexy: 300 pt; hypertension: 300 pt; having a close relative with apoplexy: 100 pt; hyperlipidemia: 100 pt; atrial fibrillation: 100 pt; and diabetes: 50 pt.

In addition, as another specific example of giving points to the risk of onset of a disease, the following literature and information on the following website are also useful.

Stroke Risk Profile: Adjustment for Antihypertensive Medication, The Framingham Study

R B D'Agostino, P A Wolf, A J Belanger and W B Kannel Stroke. 1994;25:40-43 Web information on Framingham Heart Study https://www.framinghamheartstudy.org/risk-functions/stroke/stroke.php

### (Third embodiment)

Fig. 17 will be used to describe a change in temperature as well as a change in each of a blood pressure and a pulse.

Fig. 17 illustrates a result of an experiment on how systolic blood pressure and diastolic blood pressure as well as a pulse rate of each of subjects A and B change when temperature changes from 25 to 5 degrees Celsius.

After remaining at rest sufficiently under the 25 degree Celsius environment in a seated position, the subject uses an upper arm cuff blood pressure monitor and measures the systolic blood pressure, the diastolic blood pressure and the pulse rate.

The subject thereafter enters a temperature-controlled room set at five degrees Celsius and remains at rest in the seated position for ten minutes to then measure the systolic blood pressure, the diastolic blood pressure and the pulse rate once again.

The subjects A and B both have roughly the same biological information value under the 25 degrees Celsius environment, but have different changes in the biological information under the five degrees Celsius environment since they have different biological responses against temperature. Accordingly, it is understood that the individual difference needs to be resolved though the subjects show the same disposition against the change in temperate.

Fig. 18 is a processing flowchart of an individual optimization function that resolves the individual difference.

In alarm trigger value setting, the presence or absence of a past individual alarm value matching wearer information is checked first. When the value is absent, a preset value held in a storage unit from the time of factory shipment is used as the individual alarm value.

When there is a change exceeding a predefined ambient environment condition (a change of five degrees Celsius or larger in the ambient temperature in the present example) in writing the individual alarm value, pieces of biological information before and after the change are compared to calculate the individual alarm value according to the amount of change. Moreover, the flow of the alarm value trigger setting is executed again when the individual alarm value is written.

The individual alarm value is updated in this manner by detecting the change in the individual biological information according to a specific change in the environment.

### (Fourth embodiment)

Biological information and medical information may be configured to be prepared as appropriate and adopted according to a level of priority.

Information with a high level of priority may be adopted preferentially when it exists on the basis of a table illustrated in Fig. 19, for example. The information with the high level of priority generally means one that can be an accurate piece of information in predicting a change in a risk of onset of a disease.

Information with a level of priority B or lower need not be adopted for consideration when there exists information of an upper level such as a level of priority A, whereas, when only information of a middle or low level such as the level of priority B or a level of priority C exists, each of such information is weighted and adopted.

When there exists a plurality of pieces of information with the same level of priority, information obtained on a later test date (information acquisition date) or an average value of the risk of onset of a disease calculated from each information is adopted.

An effect of environmental information on the risk of onset of a disease will be further described. While description will be provided by using only temperature and atmospheric pressure in order to simplify description, it is not limited to such information in actual implementation.

Fig. 20 is a graph illustrating a change in each of temperature and atmospheric pressure on February 12 (winter) and July 10 (summer) in 2014 in the city of Matsumoto, Nagano prefecture. As described above, the temperature and atmospheric pressure both affect the risk of onset of a disease. Specifically, a decrease in the temperature and a decrease in the atmospheric pressure each cause an increase in the blood pressure. The data is retrieved from meteorological data published by the Meteorological Agency.

Fig. 21 is a graph obtained by multiplication by a coefficient such that the risk of onset of a disease (noted as a risk factor index in the figure) caused by the temperature and atmospheric pressure illustrated in the two graphs of Fig. 20 takes a positive value.

The data on February 12 represents a typical change of winter weather in which the minimum temperature drops to ten degrees Celsius below zero while the maximum temperature is approximately three degrees Celsius. Low temperature (= high blood pressure) increases the risk of onset of a cerebral disease so that the data indicates a relatively high risk of onset of a disease throughout the day.

July 10 is a day having a small change in temperature with the minimum temperature of 22 degrees Celsius and the maximum temperature of approximately beyond 26 degrees Celsius, but the atmospheric pressure is decreased gradually throughout the day as a typhoon was actually approaching on that day. Moreover, the temperature reaches a peak around ten o'clock in the morning and then decreases till two o'clock in the afternoon. This is because the sunlight was blocked by a change in weather as well as a rainfall started at the time. Accordingly, the risk of onset of a disease increases sharply from ten o'clock in the morning to two o'clock in the afternoon. Then around three o'clock in the afternoon, the temperature rises again as the sunlight returns temporarily, followed by a rainfall once again to cause a decrease in the temperature. Accordingly, the risk of onset of a disease decreases a little at three o'clock in the afternoon and increases again thereafter.

One can thus partially know the change in the risk of onset of a disease by meteorological information alone.

While the present embodiment is described on the basis of the temperature and atmospheric pressure acquired as the environmental information, there may be used a piece of information published by a meteorological observation institution and acquired through a wireless communication unit provided in a system of the present invention.

### (Fifth embodiment)

While the pulse wave pattern and the blood pressure value are described to be correlated with each other in the first embodiment, the present embodiment further expands it to describe another biological information obtained by a pulse wave (and electrocardiogram) pattern.

Fig. 22 illustrates an overview of the pulse wave pattern. An electrocardiographic waveform (a so-called electrocardiogram) also exhibits a roughly similar form, whereby the pulse wave pattern will be described in the present embodiment assuming that the description covers the electrocardiographic waveform.

The pulse wave pattern has peaks exhibiting a characteristic shape such as a "percussion wave", a "tidal wave" and a "dicrotic wave" as illustrated in the figure. Each of these peaks generally maintains its unique shape although varying from person to person depending on a physical characteristic of a blood vessel and/or a valve function of the heart. An interval (time) of the continuous pulse wave can be measured by detecting such characteristic.

Fig. 23 is an electrocardiogram measured during approximately ten seconds. It is similar to the case with the pulse wave pattern in terms of measuring a heartbeat interval by capturing the characteristic. A so-called R-R interval in the electrocardiogram can be measured similarly by an interval of each of the "percussion wave", the "tidal wave" and the "dicrotic wave" in the pulse wave pattern but is usually measured as a P-P interval by using the "percussion wave" that is most characteristic.

This waveform is retrieved from Record 232 in MIT-BIH Arrhythmia Database published on a PhysioNet website.

It is generally rare for the heart rate to have a sudden change in the interval or a pulse dropout by a pulse unit different from a fluctuation caused by breathing or Mayer wave where, in the figure, the interval for each of a fourth pulse and a fifth pulse in particular is abnormally long as it is two to three times as long as the interval for each of a first pulse, a second pulse and a third pulse, thereby indicating a state of an irregular heartbeat.

Moreover, one can infer atrial fibrillation on the basis of small fluctuations of a baseline, disappearance of P waves and an irregular heartbeat interval in the figure.

Stagnation of blood caused by the irregular heartbeat generally becomes a factor for the occurrence of a thrombus or embolus, which eventually causes an ischemic disease. Therefore, extracting the heartbeat interval from the electrocardiographic waveform or pulse wave pattern and monitoring the value (time) of the interval as described in the first embodiment lead to grasping a sign of the ischemic disease.

Fig. 24 is a histogram obtained by measuring the heartbeat time interval for 30 minutes on the basis of the electrocardiographic waveform obtained in a similar manner. An anomaly threshold of the heartbeat time interval is set at two seconds to treat a value larger than or equal to the threshold as an anomalous state (the irregular heartbeat occurring).

It is a problem in the first place to have such anomalous state itself, but a measurer having the irregular heartbeat as a chronic disease should avoid being sensitive about an irregular heartbeat that occurs with low frequency, for example. That is, a large number of middle aged and older persons experience occurrence of the irregular heartbeat several times a day, and thus what is important is a relationship between a frequency of occurrence of the anomalous state and the heartbeat interval time being a factor for occurrence of the thrombus or embolus.

Fig. 25 is a flowchart illustrating an example of the operation to activate an ischemic cerebrovascular disease alarm.

Although detailed description will be omitted, the operation as a general description counts the occurrence of a heartbeat interval exceeding a threshold (such as two seconds) of the heartbeat interval in a predetermined period. The higher the number of occurrences, the higher the risk of onset of a disease.

Moreover, in view of the circumstances that a probability of occurrence of the thrombus and embolus increases as blood stagnates for a longer time, a more accurate risk of onset of a disease can be predicted by accumulating the heartbeat time interval, not just purely counting the number of occurrences. Moreover, in addition to simply accumulating the heartbeat time interval, the accumulation upon performing an exponential calculation (such as squaring) can contribute to the prediction of the risk of onset of a disease better matching the actual circumstances.

Fig. 26 is a graph obtained by drawing the data acquired in Fig. 25 by a method called a Lorenz plot.

Where heartbeat intervals measured successively are treated as first, second, third, ... [n-1]-th, n-th, and [n+1]-th times in order, the plot has a horizontal axis representing the heartbeat time interval of the n-th time and a vertical axis representing the heartbeat time interval of the [n+1]-th time. As described above, the heartbeat time interval does not have the disposition to change drastically by the pulse unit and is basically plotted in the vicinity of a 45-degree line.

In the case of Fig. 26, however, many points can be observed on two lines corresponding to the vertical axis = approximately 0.8 seconds and the horizontal axis = approximately 0.8 seconds. This means that the irregular heartbeat or the like occurs frequently before and after the heartbeat with a normal interval.

Fig. 27 is a histogram of heartbeat interval data of a subject without findings of a disease targeted by a certain system of the present invention and a graph applying the aforementioned Lorenz plot.

First, the histogram has a peak near 0.9 seconds with no data exceeding the threshold and can thus be said to be very normal. Moreover, most points on the Lorenz plot are plotted in the vicinity of the 45-degree line with a small number of exceptional points that fall within the threshold, whereby the data can be said to be normal.

Normality of the data is made clear by checking the histogram and the Lorenz plot in this manner.

Fig. 28 is a histogram of the heartbeat interval data of a subject with an abnormal heartbeat interval and a graph applying the Lorenz plot.

First, the histogram is roughly divided into three crests. The measurement is performed upon regulating the environment to be steady so that it is hard to imagine in a normal situation to have the heartbeat interval of 0.5 seconds or 1.3 seconds and that a failure of a valve mechanism of the heart or a disease such as the irregular heartbeat can be suspected. The value however does not exceed the threshold, whereby an anomaly cannot be detected by a determination based on the threshold. The determination can be made on the basis of the number of peaks and whether a time interval value thereof is appropriate.

Now, in the Lorenz plot, many points are plotted in a region hardly in the vicinity of the 45-degree line compared to the normal one described above. While one can well recognize the abnormality visually, the following method can be used as calculation processing to perform determination.

With the 45-degree line being y = x, an OK region (such as y = x ± 0.2 sec) is set above and below the line so that a point plotted in the region is determined as an OK plot while each of the other points is determined as an NG plot to be able to indicate the abnormality by a ratio of the numbers of these plots (the number of NG plots/the number of OK plots).

Alternatively, there is assumed a polar coordinate system obtained by converting a coordinate value xy in a Cartesian coordinate system of each point into rθ of the polar coordinate system and rotating θ counterclockwise by 45 degrees such that the 45-degree line corresponding to y = x in a direct coordinate system becomes θ = 0 in the polar coordinate system. The value of θ of an NG plot is large in the polar coordinate system so that an absolute value of θ is integrated and divided by the number of plots to obtain an average value, the magnitude of which can be used to indicate the abnormality.

The use of the Lorenz plot of the heartbeat interval as described above can also detect occurrence of the abnormal heartbeat interval and predict a change in the risk of a cerebrovascular disease.

### (Another embodiment)

While the aforementioned description has been provided assuming the case where the present invention is applied to the "person who is highly likely to have a severe disease already", it is not limited to such case.

A system of the present invention enables measurement at all times by being worn on a biological body who is a target of the measurement, and makes use of an advantage brought about by the measurement.

### (Early detection of sign of disease)

For example, even a person in his twenties who is seemingly healthy and almost free of onset of a disease has a non-zero risk of an unexpected death caused by an acute disease involving a circulatory system. Therefore, such person can also know at an early stage what kind of risk of the onset of various diseases he has by wearing the system of the present invention as well as acquiring and checking biological information. There are too many diseases to count that only require minor treatment when found at a very early stage but are difficult to treat when found late. Moreover, the person can cut down medical expenses and improve quality of life (QOL) by starting the treatment at an early stage, whereby the system contributes greatly both economically and mentally in Japan entering an aging society in the days ahead.

Furthermore, an increase in the number of persons who are conscious about proactively knowing their own health conditions by voluntarily wearing the system of the present invention causes a significant statistical difference concerning health with respect to those who are not conscious, so that an economical value of the present invention increases when those using the system receive favorable treatment in life insurance, health insurance and the like.

### (Work-related accident prevention)

The present invention can also be applied to a purpose other than the medical purposes.

The system of the present invention may be applied to a person involved in operations under a harsh work environment such as under high temperature, for example, so that various symptoms that cannot be recognized by the person himself can be perceived in advance to be able to expect an effect of reduced incidence of work-related accidents. In addition, a user can take measures against the work environment properly to be able to increase cost effectiveness of the user, which as a result contributes to improvement on economy.

### (Sporting event)

In addition, the system of the present invention can be applied to sports and training purposes.

A sport such as a marathon involving extreme straining on the body of an athlete has a high risk of onset of various acute symptoms, yet a subjective symptom does not readily appear in the first place and, at the same time, the athlete does not easily take notice of the acute symptoms due to an adrenaline effect or the like and is as a result exposed to a very dangerous environment. Moreover, the athlete having obsession for victory, one cannot expect the athlete to voluntarily request to drop out of a race and, when the athlete falls into an objectively dangerous condition to be stopped by a doctor's order to give up the race, the symptom may be severe enough that an emergency response is required of people around and that the athlete can lose his life when it takes time to transport him to a medical institution.

In this regard, the system of the present invention may be worn on the athlete in the marathon to be able to give a warning about the risk of onset of the acute symptoms on the basis of an objective index, so that the athlete himself consents to drop out of the race to be able to ensure safety of the athlete without creating psychological dissatisfaction. When the athlete uses the system of the present invention on a routine basis, a determination on the risk of onset of the acute symptoms is more appropriate (an undue warming is avoided) as the system is well customized for personal use, whereby safety can be ensured without ruining motivation of the athlete. Moreover, an increase in the number of marathon athletes wearing the system of the present invention improves efficiency of running the entire marathon race such as by reducing the number of staff in charge of medical treatment and can thus make a contribution economically. Furthermore, it may be operated to cause an athlete planning to participate in the race to wear the system of the present invention beforehand for a certain period of time and acquire biological information to be submitted to the administration of the marathon race. The risk involved in the marathon event can also be eliminated in advance by creating a mechanism that an athlete cannot enter the marathon event itself unless a level of the biological information is lower than or equal to a level at which there is a health risk.

Moreover, the stress on the athlete participating in the marathon race varies depending on various conditions including a venue (geographical information such as ups and downs and altitude of a course), temperature, humidity, wind velocity, direction of the wind, atmospheric pressure, and sunlight. The setting of a future marathon race can be improved by asking the participating athlete to submit the biological information after the race and examining a level of the risk of onset of a disease on the basis of the biological information of the athlete while considering the various conditions described above.

Moreover, a level of difficulty of the race or the course as well as an influence on the heart and lungs may be converted into a numerical form on the basis of a result of analysis on the acquired biological information of the athlete and be provided as preliminary information to a person considering participation or can be used to increase or decrease a premium of a sports insurance.

### (Heart rate when amount of activity is increased by exercise)

Calculation of a risk of onset of a disease based on a change in the heart rate at the time of an increase in the amount of activity will be described while taking as an example the time of playing a sport.

The heart rate at rest generally decreases with aging as in a result of the research conducted by Jay W. Mason. The heart rate at rest being 60 bpm or lower corresponds to a case of bradycardia and can be used as a lower limit value of the heart rate in calculating the risk of onset of a disease.

| Age group | Central value of heart rate |
|---|---|
| | |
| 0-9 | 84 |
| | |
| 10-19 | 70 |
| | |
| 20-29 male | 63 |
| | |
| 20-29 female | 69 |
| | |
| 30-69 | 68 |
| 70-99 | 65 |

Note however that the heart rate at rest of a person with a finding of athlete's heart common in sports athletes and enthusiasts (such as marathon and bicycle) requiring endurance is around 30 to 50 [beats/minute]. It is at a level at which an ordinary person is diagnosed with sinus bradycardia, but is a result of increased cardiac output efficiency achieved by training and needs to be taken into consideration at the time of calculating the risk of onset of a disease.

Moreover, it is reported that a person doing aerobic exercise with moderate exercise stress such as walking and swimming on a continuing basis has a lower value of carotid-femoral pulse wave velocity (cfPWV) being the index for the degree of arteriosclerosis than a person not doing such exercise (Kakiyama T, et al. angiology 1998). Furthermore, the person not doing the exercise has improved cfPWV by doing the aerobic exercise for approximately several weeks, so that such fact needs to be taken into consideration at the time of calculating the risk of onset of a disease by referring to an exercise history stored in a storage unit of a device of the present invention.

Fig. 29 illustrates a result of actual measurement of a change in the heart rate of three subjects at the time of the increase in the amount of activity.

The increase in the amount of activity is achieved by a stepping exercise with the height of 23 cm and rhythm of 30 [steps/minute].

Here, the system of the present invention includes therein one or more of an acceleration sensor, a myogenic potential sensor, a bioimpedance sensor and the like, for example, and detects the increase in the amount of activity. The heart rate starts increasing immediately after the increase in the amount of activity and, although varying among individuals, the rate of increase slows down after about two minutes and reaches a saturated state after about five minutes. An alarming threshold is updated successively along this change. The onset of paroxysmal supraventricular tachycardia or ventricular tachycardia is suspected when the heart rate increases at rest, but a possibility of the onset can be eliminated on the basis of a relationship between the heart rate and the amount of activity.

As a preferable example of use of the system of the present invention, when the system detects a fluctuation in the level of the heart rate at the time of the increase in the amount of activity or a state in which the heart rate is undetectable during a marathon race or cycling event, for example, a wearer, medical personnel or an organizer is notified of the detected information to be able to use the information to determine whether to stop the event or necessity to use AED.

Moreover, the change in the heart rate varies among the three subjects asked to do the same exercise due to individual differences. A threshold for issuing an alarm on the basis of the difference is optimized for the wearer from a preset value, whereby appropriateness of notification of the risk of onset of a disease by the system of the present invention is increased.

The "time of the increase in the amount of activity" used herein is defined as a state in which all elements (muscles, brain, nerves, circulatory organs, respiratory organs, digestive organs, sensory organs, excretory organs, reproductive organs and the like) making up a human or an animal perform an activity using correspondingly more energy than at rest.

### (Risk of relapse of disease)

Moreover, in the present embodiment, there will be described calculation of a risk of onset of a disease of a wearer with a previous history of apoplexy.

According to a result obtained from the Akita stroke registry (The Japan Geriatrics Society Magazine, 2008; 45: 169-171), approximately 12% of those with the history of a disease experiences a relapse, where there is a high rate of the relapse of the same disease as an initial disease. Therefore, it needs to be treated as a risk factor for increasing the risk of onset of the disease.

A main problem to be solved in the present embodiment is that existing effort and technology alone do not lead to a sufficient increase in awareness of the risk or an avoidance behavior for the risk factor of a person with a history of the disease regardless of the obstruction in life caused by long-term recuperation, an after effect of the disease and the like by the onset thereof once. Accordingly, when worn on a wearer with a previous history, the system acquires through an information input unit (1) time of onset of a disease, (2) a specific type of the disease and an affected site, (3) an after effect, and (4) presence or absence of medication (an antithrombotic drug or antihypertensive drug) relevant to apoplexy, which are then used as medical information to improve accuracy of calculating the risk of onset of the disease.

### (Handling of irregular heartbeat)

In general, the respiration rate at rest is around 15 to 20 [breaths/minute] where the value of an R-R interval is detected as a peak of approximately 0.3 to 0.5 Hz in accordance with a breathing cycle by a frequency analysis on the interval value. Unlike other irregular heartbeats, the respiratory sinus arrhythmia is a normal biological response and is thus distinguished from a change in the R-R interval caused by a bradycardia - tachycardia syndrome, atrial fibrillation, ventricular tachycardia, torsades de pointes, an atrioventricular block, a premature ventricular contraction or a premature supraventricular contraction and excluded from the factor for increasing the risk of onset of a disease.

### (Issuing alarm)

An alarm of the system of the present invention will now be described.

An alarm output unit in the system of the present invention can employ several methods.

A target to which the alarm is issued can be broadly classified as a subject person (or an animal) wearing the system and a third party (a medical institution, a relative, a specific person, or an unspecified person around of whom one wishes to request rescue or aid).

### (Alarm to concerned party)

The alarm is switched from a minor alarm to a major alarm in order according to severity. The minor alarm in this case means an alarm that can be reached to a relatively narrow range, and the major alarm in contrast means one that can be reached to a relatively wide range.

When issued in the form of sound, the alarm changes from one at a low volume to one at a high volume. Besides sound, there can be applied light, an oscillation, heat, a scent, taste and the like. In the case of light, a difference can be set by a wavelength and a release pattern in addition to intensity. In the case of an oscillation, a difference can be set by the waveform itself of an oscillating wave, an oscillating pattern and the like in addition to intensity. In the case of a scent, perfume raw material in various perfumed capsules may be vaporized by heating or reducing pressure to flow to the vicinity of a nostril of the wearer, where it is needless to say that a difference can be set by the type of the scent and intensity of stimulation to an olfactory cell. In the case of taste, a flavoring solution may be stored in an artificial tooth provided with a valve mechanism that is operated by a signal such as a radio wave from the outside so that the solution can be flown into an oral cavity (especially near a tongue) in response to the alarm or that a difference can be set according to the amount of the solution flowing into the oral cavity, while at the same time a dose of medicine to be taken only once may be contained in the solution itself to allow for more effective handling. For a person with an irregular heartbeat, for example, an antiarrhythmic drug or a thrombosis prophylactic drug is suitable as the dose of medicine to be taken only once.

The alarm issued in the form of voice may inform a concerned party wearing the system of not only the type of the alarm but also a cause of the alarm and a proposal for an avoidance behavior. This allows the concerned party to understand a fact that the risk of onset of a disease is increased as well as the cause thereof and the proposal for the avoidance behavior to be able to handle the situation calmly and reliably. Moreover, when the concerned party falls unconscious or the like by misfortune before performing the avoidance behavior, one can expect a third party who happens to be around at the time to handle the situation, the third party apprehending the alarm issued in the form of voice and contacting a medical institution on behalf of the concerned party or the like.

The aforementioned alarm issued in the form of voice may be replaced by wireless or wired communication. A general-purpose electronic terminal can receive an alarm issued through communication when a de facto standard communication protocol becomes widespread, in which case one can know the detail of the alarm, a cause thereof and a proposal for an avoidance behavior more promptly and reliably than the alarm in the form of voice.

With such device included as standard in an emergency vehicle such as an ambulance, when the concerned party experiencing the onset of a disease and falling unconscious by misfortune is carried into the emergency vehicle, one can immediately grasp information such as the detail of the alarm, the cause thereof and the proposal for the avoidance behavior to be able to quickly start first-aid treatment and accurately select a hospital to which the concerned party is taken. It is needless to say at this time that various pieces of information including biological information of the concerned party and environmental information present within the system of the present invention can be used in an integrated manner.

Moreover, it is required that the alarm issued to the concerned party can be clearly recognized in terms of disease prevention. Depending on the circumstances, however, a limitation needs to be placed on the alarm as with a ring tone of a mobile phone.

In a quiet environment such as a theater or museum, for example, the clear alarm is to be avoided as much as possible since it can be a false alarm though the alarm is used to avoid a danger of one's life. In such case, it may be adapted to be able to switch at will a destination of an alarm issued to a third party to be described. That is, for example, the wearer may declare his disease to an attendant at the time of reception in the theater to switch setting such that the alarm issued to the third party is sent to a receiver set up in the theater (and the alarm to the wearer himself is moderated). Accordingly, when the risk of onset of a disease of the wearer is increased by misfortune, the theater attendant paying attention to the alarm in place of the wearer himself can guide him from inside the theater to a greenroom or the like to not disturb the other audience, whereby the wearer and the other audience can continue to enjoy a play while maintaining the atmosphere of the theater. This can also be used as a fail-safe in the event the concerned party does not notice the alarm due to a sound effect and/or a lighting effect of the theater.

Furthermore, a different method can be employed in an environment in which vision and hearing are limited.

When an alarm is to be issued to a person doing scuba diving or a spacewalk, for example, an alarm in the form of sound, light or oscillation is not easily noticed and is inappropriate. In such case, there may be employed a method of providing an aroma generator for alarming together with an oxygen tank. The aroma generator generates an aromatic component such as citrus aroma that is clearly perceivable and then mixes it with oxygen supplied from the oxygen tank. The person in the middle of doing scuba diving or the spacewalk can use a sense of smell to be able to notice an abnormality in himself by configuring the aroma generator to be actuated when the risk of onset of a disease is increased.

In addition, when the wearer is a person (VIP) such as a politician or a business entrepreneur having an influence on the world, it is a problem for an alarm issued to the VIP to be recognized by a third party. In other words, it is tantamount to exposing to the world that the person has concerns in terms of health. When such VIP becomes the wearer, an alarm may be output secretly to an electronic terminal of a secretary or an SP by the VIP. The person having the electronic terminal may implicitly tell the VIP that the alarm is issued to be able to prevent the onset of a disease, whereby the VIP does not need to expose to the third party the secret being the concern about his health.

### (Alarm to third party)

The alarm output to the third party will now be described.

As described above, the alarm output unit outputting the alarm about the increase in the risk of onset of a disease targets not just the person (and animal) wearing the system of the present invention. The alarm output unit assumes a case where the wearer suddenly experiencing an increase in the risk of onset of a disease has an attack and falls unconscious before being able to perform the avoidance behavior, or a case where the wearer (or the animal) is in a state unable to perform the avoidance behavior in the first place (such as when the wearer is a newborn infant, an elderly person with dementia, or a hospitalized patient in the ICU). In such case, the alarm is issued to the third party, who receives the alarm to aid the avoidance behavior. When the wearer has some kind of problem in communicating his information due to clouding of consciousness, loss thereof, or a problem with a physical function, the third party can refer to information in a storage unit of the device of the present invention and use the information for determining what kind of treatment is to be performed on the wearer.

The third person corresponds to a specific person such as one at a medical institution, a relative, a friend, an acquaintance, a neighbor, a welfare commissioner or the like and an unspecified person who happens to be around at the time of the onset of a disease.

It is assumed that the specific person is not present in the vicinity of the wearer, in which case the wireless communication (using the radio wave, light or ultrasound) is employed. The wired communication can also be employed when a movement of the wearer is limited.

The unspecified person who happens to be around at the time of the onset of a disease may employ a method similar to that using sound or light among the alarms issued to the concerned party.

### (Application to animal)

There has been illustrated the embodiment regarding the biological information of a person as the subject, which however is not limited to the person. The biological information or the like of an animal can also be measured continuously at all times in a similar manner by making use of an advantage that the system can be worn on a living body of the subject of measurement.

### (Racehorse)

The system of the present invention can be applied for the purpose of knowing a cardiopulmonary function and/or administering health of a racehorse, for example.

As with a person, it is important to know the health condition of the racehorse running under severe stress.

### (Wild animal)

The system of the preset invention can also be applied for the purpose of unraveling the ecology of a wild animal.

While an ecological survey on many animals is conducted at a specific site such as a zoo, a survey allowed on the wild animal has been little more than a behavioral survey using a hidden camera installed at a specific spot so that biological information obtained under an actual habitat condition is more valuable for a zoologist or the like. In a research on how the temperature and heart rate of a bear change before and after hibernation, for example, one can obtain a measurement that is high in accuracy and closer to one in the actual condition.

### (Monitoring pet)

Next, there will be provided description regarding the device of the present invention worn on a pet, primarily a dog or cat.

There is already put to practical use a device and a service that transfer, to a web terminal such as a PC, a video or still image obtained by a camera installed in a room where the pet lives and allow one to check the condition of the pet in an empty home. However, with the image alone, it is difficult to determine whether an animal to be monitored is asleep or in an abnormal state (having an attack, being stiff or dead). Accordingly, the system of the present invention can be applied to be able to successively acquire biological information and environmental information of the pet being the animal to be monitored and notify a raiser of the abnormal state to enable treatment at a stage before the animal falls into a severe condition. The information can also be linked to an apparatus inside the empty home such as an air conditioner, an automatic feeder, a curtain opening/shutting apparatus to be able to be used for improving the environment of the animal to be monitored.

### (Monitoring livestock)

In addition, there will be described application of the system of the present invention to a pastured livestock animal.

The livestock animal is pastured mainly in summertime. At this time, one is unable to grasp the conditions of all the livestock animals due to the large area of a pastureland and the large number of the animals, thereby resulting in a delay in finding out stealing, a disease, falling or predation by a carnivore such as a bear of the livestock animal when such case occurs. Accordingly, the system of the present invention can be applied to be able to successively acquire biological information and environmental information of the livestock animal being monitored and detect, for example, a cardiopulmonary abnormality caused by a disease, a change in posture caused by falling off a hill or cliff, and a stress condition and a movement of the body at the time of an attack by the carnivore to be able to minimize the damage by informing a person in charge of the detection.

While the present invention has been described with the preferred embodiments, it is needless to say that the present invention is not limited to these embodiments but can be modified in many ways without departing from the spirit of the invention.

### {Industrial Applicability}

According to the biological information reading unit that can be mass produced and the system of the present invention including the biological information reading unit, the biological information can be measured continuously at all times without imposing physical and psychological burdens on the measurer so that the change in the risk of onset of a severe disease can be predicted accurately on the basis of the measured biological information, which may be applied to a purpose other than the medical purpose to be able to expect efficient training and operation by issuing an appropriate alarm in terms of safety or the like to an athlete playing a sport that requires straining and a worker under a severe environment.

### {Reference Signs List}

No reference signs

## Claims

1. A system for predicting a risk of onset of a cerebrovascular disease, the system comprising:
a biological information acquisition unit for continuously acquiring pulse wave information as biological information of a subject;
an acquired information storage unit for storing the pulse wave information acquired by the biological information acquisition unit;
a disease onset risk prediction unit for predicting a risk of onset of a cerebrovascular disease of the subject on the basis of the pulse wave information being stored; and
an alarm output unit for issuing an alarm about a risk of onset of a disease on the basis of the prediction by the disease onset risk prediction unit, the disease onset risk prediction unit predicting the risk of onset of the cerebrovascular disease on the basis of a change in each of a blood pressure and a pulse wave pattern that are based on the pulse wave information acquired continuously.

2. The system for predicting a risk of onset of a cerebrovascular disease according to claim 1, wherein
the prediction of the risk of onset of a disease is to determine whether or not the change in each of the blood pressure and the pulse wave pattern being acquired exceeds a normal range, and
the system issues an alarm when the change exceeds the normal range.

3. The system for predicting a risk of onset of a cerebrovascular disease according to claim 2, wherein
the determination on whether or not the change in each of the blood pressure and the pulse wave pattern exceeds the normal range is made by employing a statistical determination method that determines whether the change in each of the blood pressure and the pulse wave pattern being acquired is in the normal range or an abnormal range.

4. The system for predicting a risk of onset of a cerebrovascular disease according to claim 3, wherein
the system determines that the risk of onset of a disease is high when a blood pressure value and a pulse rate acquired from the subject deviate to a large extent from a range of correlation, on the basis of a correlation between the blood pressure and the pulse rate acquired from the pulse wave pattern.

5. The system for predicting a risk of onset of a cerebrovascular disease according to any one of claims 1 to 4, wherein
one or a plurality of pieces of information among activity information, physical information, disease information and environmental information of the subject is acquired beforehand, and
the onset risk prediction unit predicts the risk of onset of a cerebrovascular disease on the basis of all or an arbitrarily selected piece of the activity information, the physical information, the disease information and the environmental information of the subject as well as the pulse wave information being acquired.

6. The system for predicting a risk of onset of a cerebrovascular disease according to any one of claims 1 to 5, wherein
the cerebrovascular disease to be predicted includes an ischemic cerebrovascular disease and a hemorrhagic cerebrovascular disease.
